# EUROPÄISCHE PATENTSCHRIFT

(11) **EP 1 234 056 B1**
(45) Veröffentlichungstag und Bekanntmachung des Hinweises auf die Patenterteilung: **07.12.2005**
(21) Anmeldenummer: 00979641.8
(22) Anmeldetag: 29.11.2000
(51) Int. Cl.: C12Q 1/68

(54) **DYNAMISCHE BESTIMMUNG VON ANALYTEN DURCH ARRAYS AUF INNEREN OBERFLÄCHEN**
DYNAMIC DETERMINATION OF ANALYTES USING ARRAYS ON INTERNAL SURFACES
DETERMINATION DYNAMIQUE D'ANALYTES EN UTILISANT UNE PUCE LOCALISEE SUR UNE SURFACE INTERNE

(30) Priorität: 29.11.1999 DE 19957319
(43) Veröffentlichungstag der Anmeldung: 28.08.2002
(73) Patentinhaber: febit AG, 68167 Mannheim (DE)
(72) Erfinder: STÄHLER, Peer, F., 68167 Mannheim (DE); STÄHLER, Cord, F., 69469 Weinheim (DE); SCHLAUERSBACH, Andrea, 64291 Darmstadt (DE); MÜLLER, Manfred, 80689 München (DE); BAUM, Michael, 69124 Heidelberg-Kirchheim (DE); BEIER, Markus, 69121 Heidelberg (DE)
(74) Vertreter: Weiss, Wolfgang
(86) Internationale Anmeldenummer: PCT/EP2000/011968
(87) Internationale Veröffentlichungsnummer: WO 2001/040509

(56) Entgegenhaltungen:
- WO-A-95/09248
- WO-A-99/39004
- US-A- 5 683 881
- US-A- 5 871 928

## Beschreibung

Die Erfindung betrifft ein Verfahren zur Bestimmung von Analyten unter Verwendung von Trägern, die Arrays von unterschiedlichen Rezeptoren in immobilisierter Form auf ihrer Oberfläche enthalten. Das Verfahren wird dynamisch in mehreren Zyklen durchgeführt, wobei die aus einem vorhergehenden Zyklus gewonnene Information zur Modifizierung bzw. Veränderung der Rezeptoren im nachfolgenden Zyklus genutzt wird.

### 1. Einleitung

Für die Grundlagenforschung, die Medizin die Biotechnologie sowie weitere wissenschaftliche Disziplinen ist die Erfassung biologisch relevanter Information in definiertem Untersuchungsmaterial von herausragender Bedeutung. Zumeist steht dabei die genetische Information im Mittelpunkt des Interesses. Diese genetische Information besteht in einer enormen Vielfalt unterschiedlicher Nukleinsäuresequenzen, der DNA. Die Nutzung dieser Information im biologischen Organismus führt über die Herstellung von Abschriften der DNA in RNA meist zur Synthese von Proteinen. Weitere wertvolle Information kann aus der Analyse von RNA und Proteinen sowie den anfallenden Stoffwechselprodukten gewonnen werden.

Um die Wirkprinzipien der Natur auf Basis der Genetik besser verstehen zu können, ist eine effiziente und sichere Entschlüsselung von DNA-Sequenzen notwendig. Die Detektion von Nukleinsäuren und die Bestimmung der Abfolge der vier Basen in der Kette der Nukleotide, die generell als Sequenzierung bezeichnet wird, liefert wertvolle Daten für Forschung und angewandte Medizin. In der Medizin konnte in stark zunehmendem Maße durch die in vitro-Diagnostik (IVD) ein Instrumentarium zur Bestimmung wichtiger Patientenparameter entwickelt und dem behandelnden Arzt zur Verfügung gestellt werden. Für viele Erkrankungen wäre eine Diagnose zu einem ausreichend frühen Zeitpunkt ohne dieses Instrumentarium nicht möglich. Hier hat sich die genetische Analyse als wichtiges neues Verfahren etabliert.

In enger Verzahnung von Grundlagenforschung und klinischer Forschung konnten die molekularen Ursachen und (pathologischen) Zusammenhänge einiger Krankheitsbilder bis auf die Ebene der genetischen Information zurückverfolgt und aufgeklärt werden. Diese wissenschaftliche Vorgehensweise steht jedoch noch am Anfang ihrer Entwicklung und gerade für ihre Umsetzung im Rahmen von Therapiestrategien bedarf es stark intensivierter Anstrengungen. Insgesamt haben die Genomwissenschaften und die damit im Zusammenhang stehende Nukleinsäureanalytik sowohl zum Verständnis der molekularen Grundlagen des Lebens als auch zur Aufklärung sehr komplexer Krankheitsbilder und pathologischer Vorgänge wichtige Beiträge geleistet.

### 2. Stand der Technik

Weitere wesentliche Beiträge durch molekulare Analyseverfahren sind sowohl für die Entwicklung von Therapien und Wirksubstanzen im Umfeld von Medizin als auch für die Entwicklung biotechnischer Ansätze zu erwarten. Solche gehören z.B. zu den Bereichen Rohstoffe, Umwelt, Produktionsverfahren, Agrar und Nutztierzucht oder Forensik.

Genetische Information wird durch Analyse von Nukleinsäuren, meist in Form von DNA, gewonnen. Es gibt drei wesentliche Techniken für die Analyse von DNA. Der wichtigste Vertreter der ersten Kategorie ist die Polymerase-Kettenreaktion (PCR). Diese und verwandte Methoden dienen der selektiven enzymgestützen Vervielfältigung (Amplifikation) von Nukleinsäuren, indem kurze flankierende Stränge mit bekannter Sequenz genutzt werden, um die enzymatische Synthese des dazwischenliegenden Bereiches zu starten, meist mittels einer Polymerase. Dabei muß die Sequenz dieses Bereiches nicht im Detail bekannt sein. Der Mechanismus erlaubt damit anhand eines kleinen Ausschnittes an Information (den flankierenden DNA Strängen) die selektive Vervielfältigung eines bestimmten DNA Abschnittes, so dass dieser vervielfältigte DNA Strang in großer Menge für weitere Arbeiten und Analysen zur Verfügung steht.

Als zweite Basistechnik wird die Elektrophorese verwendet. Dabei handelt es sich um eine Technik zur Trennung von DNA Molekülen anhand ihrer Größe. Die Trennung erfolgt in einem elektrischen Feld, das die DNA Moleküle zur Wanderung zwingt. Durch geeignete Medien, wie z.B. vernetzte Gele, wird die Bewegung im elektrischen Feld abhängig von der Molekülgröße erschwert, so dass kleine Moleküle und damit kürzere DNA Fragmente schneller wandern als längere. Elektrophorese ist die wichtigste etablierte Methode für die DNA Sequenzierung und darüber hinaus für viele Verfahren zur Reinigung und Analyse von DNA. Das verbreitetste Verfahren ist die Flachbett-Gelelektrophorese, die im Bereich der Hochdurchsatzsequenzierung allerdings zunehmend von der Kapillar-Gelelektrophorese verdrängt wird.

Bei der dritten Methode handelt es sich um die Analyse von Nukleinsäuren durch sogenannte Hybridisierung. Hierbei wird eine DNA Sonde mit bekannter Sequenz verwendet, um eine komplementäre Nukleinsäure zu identifizieren, meistens vor dem Hintergrund eines komplexen Gemisches von sehr vielen DNA- oder RNA-Molekülen. Die passenden Stränge binden sich stabil und sehr spezifisch aneinander.

Die drei Basistechniken kommen häufig in Kombination vor, indem z.B. das Probenmaterial für ein Hybridisierungsexperiment vorher selektiv durch PCR vervielfältigt wird.

Bei der Sequenzanalyse auf einem DNA-Trägerchip nutzt man ebenfalls das Prinzip der Hybridisierung von zueinander passenden DNA-Strängen aus. Die Entwicklung von DNA-Trägerchips oder DNA-Arrays bedeutet eine extreme Parallelisierung und Miniaturisierung des Formats von Hybridisierungs-experimenten. DNA in einer Probe kann nur an den Stellen an die auf dem Träger fixierte DNA binden, an denen die Sequenz der beiden DNA-Stränge übereinstimmt. Mit Hilfe der fixierten DNA auf dem Chip kann selektiv die komplementäre DNA in der Probe nachgewiesen werden. Dadurch werden beispielsweise Mutationen im Probenmaterial durch das Muster erkannt, das nach der Hybridisierung auf dem Träger entsteht.

Der wesentliche Engpass bei der Bearbeitung von sehr komplexer genetischer Information mit einem solchen Träger ist der Zugriff auf diese Information durch die begrenzte Zahl von Messplätzen auf dem Träger. Ein solcher Messplatz ist ein Reaktionsbereich, in dem bei der Herstellung des Trägers DNA-Moleküle als spezifische Reaktionspartner, sog. Sonden, synthetisiert werden.

Für einen größeren Datendurchsatz gibt es prinzipiell zwei Möglichkeiten: Die eine besteht darin, die Anzahl der Messplätze auf einem Reaktionsträger zu erhöhen. Die Anzahl der möglichen Sonden bleibt aber immer noch niedrig im Vergleich zur biologischen Vielfalt und mimimal im Verhältnis zur statistischen Vielfalt. Die zweite beruht darauf, die Anzahl der unterschiedlichen Sonden zu steigern, die das System pro Zeit (und pro eingesetztem Geld) erzeugen und für Hybridisierung bereitstellen kann. Die zweite Möglichkeit hat etwas mit der Anzahl an Varianten zu tun, die im System generiert und für die Analyse zur Verfügung gestellt werden (Datendurchsatz).

Bei dem Begriff genetische Information muss unterschieden werden zwischen unbekannten Sequenzen, die zum ersten mal dekodiert werden (dies wird im allgemeinen unter dem Begriff Sequenzieren verstanden, auch *de novo* Sequenzierung) und bekannten Sequenzen, die aus anderen Gründen als dem erstmaligen Dekodieren identifiziert werden sollen. Solche anderen Gründe sind beispielsweise die Untersuchung der Expression von Genen oder die Verifizierung der Sequenz eines interessierenden DNA Abschnittes bei einem Individuum. Dies kann z.B. geschehen, um die individuelle Sequenz mit einem Standard zu vergleichen, wie bei der Mutationsanalyse von Krebszellen und der Typisierung von HIV Viren.

Für die *de novo* Sequenzierung werden bislang fast ausschließlich elektrophoretische Methoden verwendet. Am schnellsten ist die Kapillarelektrophorese.

Träger spielen für die *de novo* Sequenzierung bislang kaum eine Rolle. Dies liegt an prinzipiellen Limitationen: für den Informationsgewinn durch Sequenzvergleich müssen Sonden auf dem Träger bereitgestellt werden. Bei der Bearbeitung von unbekanntem Material braucht man sehr viele unterschiedliche Sonden (Varianten). Kein bislang bekanntes Verfahren ist in der Lage, die notwendigen Varianten-Zahlen für ein effektives Sequenzieren durch Sequenzvergleich von sehr großen DNA Mengen zu generieren. Solche sehr großen DNA Mengen liegen z.B. bei der Sequenzbestimmung von ganzen Genomen vor.

Bislang sind im Wesentlichen zwei Verfahren zur Herstellung von Trägern bekannt. Beim ersten Herstellungsverfahren werden die fertigen Sonden einzeln entweder in einem Synthesizer (chemisch) oder aus isolierter DNA (enzymatisch) hergestellt und diese dann in Form winziger Tropfen auf die Oberfläche des Träger aufgebracht, und zwar jede einzelne Sorte der Sonden auf einen einzelnen Messplatz. Das verbreitetste Verfahren hierzu leitet sich aus der Tintenstrahldrucktechnik ab, daher werden diese Verfahren unter dem Oberbegriff Spotting zusammengefaßt. Ebenfalls weit verbreitet sind Verfahren mit Nadeln. Nur durch die Mikro-Positionierung von Druckkopf oder Nadel kann später ein Signal auf dem Chip einer bestimmten Sonde zugeordnet werden (Array mit Zeilen und Spalten). Entsprechend genau müssen die Spotting-Geräte arbeiten.

Bei der zweiten Methode werden die DNA Sonden direkt auf dem Chip hergestellt, und zwar durch ortsspezifische Chemie *(in situ* Synthese). Dazu gibt es derzeit zwei Verfahren.

Das eine arbeitet mit den oben beschriebenen Spotting-Geräten, jedoch mit dem Unterschied, dass die winzigen Tropfen entsprechende Synthesechemikalien enthalten, so dass durch die Mikro-Positionierung dieser Chemikalien die ortsaufgelöste Chemie betrieben werden kann. Die Technologie erlaubt eine beliebige Programmierung der Sequenz der entstehenden Sonden. Allerdings ist bisher der Durchsatz, das heißt die Anzahl der Sonden pro Zeit, nicht wirklich hoch genug, um große Mengen genetischer Information umzusetzen, zudem ist die Größe der Meßplätze begrenzt.

Sehr viel mehr Messplätze pro Zeit lassen sich mit der zweiten Methode herstellen: die parallele Synthese der Sonden mit einer lichtabhängigen Chemie. Damit wurden bereits über 100.000 Messplätze pro Chip in wenigen Stunden synthetisiert.

Das Verfahren wird mit zwei technischen Lösungen für die Belichtung betrieben. Die eine verwendet photolithographische Masken und erzeugt durch die hoch entwickelte Optik sehr viele Messplätze auf dem DNA-Träger. Allerdings ist die Wahl der Sondensequenz sehr limitiert, da entsprechende Masken hergestellt werden müssen. Für das erfindungsgemäße Verfahren ist diese Herstellmethode daher wenig geeignet. Wesentlich aussichtsreicher sind Verfahren mit frei programmierbarer Sondensequenz, die auf Basis entsprechend steuerbarer Lichtquellen arbeiten. Solche Herstellverfahren für Sonden auf einem Träger sind u.a. in den Patentanmeldungen DE 198 39 254.0, DE 198 39 256.7, DE 199 07 080.6, DE 199 24 327.1, DE 199 40 749.5, PCT/EP99/06316 und PCT/EP99/06317 beschrieben.

Zusammenfassend läßt sich sagen, dass mit den bisher etablierten Techniken zur Bearbeitung größerer Mengen genetischer Information mit ganz oder teilweise unbekannter Zusammensetzung, nämlich Elektrophoreseverfahren und Biochip-Trägern, eine Limitation des Durchsatzes gegeben ist. Hochdurchsatzprojekte für die Neusequenzierung sind bisher auf Größensortierung mit Elektrophorese angewiesen (u.a. das Human Genom Projekt HUGO). Hier sind zwar Verbesserungen durch Miniaturisierung und Parallelisierung zu erwarten, aber keine Durchbrüche, da die Technik an sich nicht verändert werden kann. Elektrophorese kann die meisten Anwendungen von Biochips, wie z.B. Expressions-Muster oder Mutations-Screening, nicht oder nur sehr viel langsamer leisten. Die bisher bekannten Biochips sind ihrerseits für Neusequenzierung ungeeignet, der Schwerpunkt liegt auf der hochparallelen Bearbeitung von Material auf Basis bekannter Sequenzen (u.a. in Form von synthetischen Oligonukleotiden als Sonden). Eine dynamische bzw. evolutive Auswahl, ein Informationszyklus oder ein Selektionsprozess, können von diesen Biochips nicht in effizienter und ökonomischer Weise geleistet werden. Beide Formate haben einen limitierten Durchsatz an genetischer Information. Um diesen Durchsatz zu erhöhen müssen neue Ansätze entwickelt werden. Das erfindungsgemäße Verfahren ist ein solcher Ansatz, der für Nukleinsäuren, aber auch für andere Substanzklassen wie Peptide, Proteine und andere organische Moleküle eingesetzt werden kann.

US-Patent 5,683,881 offenbart ein iteratives Sequenzierungsverfahren, umfassend einen ersten Hybridisierungszyklus mit einem ersten Array und weitere Hybridisierungszyklen mit weiteren Arrays mit verlängerten Oligonukleotiden, die auf der Basis der aus dem vorangegangenen Zyklus erhaltenen Ergebnisse hergestellt wurden. Das dort offenbarte Verfahren verwendet jedoch kein integriertes Synthese- und Analyseverfahren oder einen geschlossenen Träger.

WO 99/39004 offenbart eine iterative Methode zur Sequenzierung von grundsätzlich bekannten Sequenzen. Auch hier wird kein integriertes Synthese- und Analyseverfahren mit einem geschlossenen Träger verwendet.

### 3. Gegenstand der Erfindung

Die Erfindung betrifft ein Verfahren zur Bestimmung von Analyten in einer Probe umfassend die Schritte:
(a) Durchführen eines ersten Bestimmungszyklus umfassend:
   (i) Bereitstellen eines geschlossenen Trägers mit einer Oberfläche auf einer innen liegenden Struktur, die an einer Vielzahl von vorbestimmten Bereichen immobilisierte Rezeptoren enthält, wobei die Rezeptoren in einzelnen Bereichen auf dem Reaktionsträger jeweils eine unterschiedliche Analytspezifität aufweisen,
   (ii) Inkontaktbringen der Probe, die zu bestimmende Analyten enthält, mit dem Träger unter Bedingungen, bei denen eine Bindung zwischen den zu bestimmenden Analyten und dafür spezifischen Rezeptoren auf dem Träger erfolgen kann, und
   (iii) Identifizieren der vorbestimmten Bereiche auf dem Träger, an denen eine Bindung in Schritt (ii) erfolgt ist,
(b) Durchführen eines nachfolgenden Bestimmungszyklus umfassend:
   (i) Bereitstellen eines weiteren geschlossenen Trägers mit einer Oberfläche auf einer innen liegenden Struktur, die an einer Vielzahl von vorbestimmten Bereichen immobilisierte Rezeptoren enthält, wobei die Rezeptoren in einzelnen Bereichen auf dem Reaktionsträger jeweils eine unterschiedliche Analytspezifität aufweisen, wobei für den weiteren Träger Rezeptoren ausgewählt werden, bei denen in einem vorhergehenden Zyklus ein vorbestimmtes charakteristisches Signal, das für die Bindung des Analyten an einen spezifischen Rezeptor charakteristisch ist, beobachtet worden ist, und wobei die ausgewählten Rezeptoren oder/und die Bedingungen der Rezeptor-Analyt-Bindung gegenüber einem vorhergehenden Bestimmungszyklus verändert werden,
   (ii) Wiederholen von Schritt (a) (ii) mit dem weiteren Träger und
   (iii) Wiederholen von Schritt (a) (iii) mit dem weiteren Träger und
(c) gegebenenfalls Durchführen von einem oder mehreren weiteren nachfolgenden Bestimmungszyklen jeweils mit Auswahl und Veränderung der Rezeptoren gemäß Schritt (b) (i), bis eine ausreichende Information über die zu bestimmenden Analyten vorliegt oder/und bis das Signal nach erfolgtem Bindungsereignis einem vorbestimmten Kriterium entspricht, wobei die Durchführung von Bestimmungszyklen die integrierte Herstellung und Auswertung eines Trägers in einem Gerät umfasst ist.

Unter Träger oder Reaktionsträger sollen in diesem Zusammenhang geschlossene Träger verstanden werden. Offene Träger können planar (z.B. Labordeckglas), aber auch speziell geformt (z.B. schalenförmig) sein. Bei allen offenen Trägern ist als Oberfläche eine Fläche auf der Außenseite des Trägers zu verstehen. Geschlossene Träger haben eine innenliegende Struktur, die beispielweise Mikrokanäle, Reaktionsräume oder/und Kapillaren umfaßt. Hier sind als Oberflächen des Trägers die Oberflächen von zwei- oder dreidimensional ausgeprägten Mikrostrukturen im Inneren des Trägers zu verstehen. Natürlich ist auch die Kombination von innenliegenden geschlossenen und außenliegenden offenen Oberflächen in einem Träger denkbar. Als Materialien für Träger kommen beispielweise Glas wie Pyrex, Ubk7, B270, Foturan, Silizium und Siliziumderivate, Kunststoffe wie PVC, COC oder Teflon sowie Kalrez zum Einsatz.

Eine flexible, schnelle und voll automatische Methode der Array-Generierung mit integrierter Detektion in einem logischen System, wie sie z.B. DE 199 24 327.1, DE 199 40 749.5 und PCT/EP99/06317 beschrieben ist, ermöglicht es, innerhalb von kurzer Zeit durch die Auswertung der Daten eines Arrays die notwendigen Informationen für den Aufbau eines neuen Arrays zu erhalten (Informationszyklus). Dieser Informationszyklus erlaubt eine automatische Anpassung der nächsten Analyse durch Auswahl geeig-neter Polymersonden für das neue Array. Dabei kann unter Berücksichtigung des erhaltenen Ergebnisses die Breite der Fragestellung zugunsten einer höheren Spezifität eingeschränkt oder die Richtung der Fragestellung moduliert werden. Weiterhin können durch die Veränderung von Rezeptoren auch teilspezifische Analytbindungen, z.B. Bindungen von einander "ähnlichen" Analytgruppen verfolgt werden, bis eine genaue Zuordnung des Analyten in der Probe möglich ist. Somit wird im Vergleich zu den bisher gängigen und oben teilweise beschriebenen Verfahren mit relativ geringem Aufwand ein Vielfaches der bisherigen Informationsmenge umgesetzt und dabei wertvolle Information gesammelt.

Bei dem erfindungsgemäßen Verfahren wird dieses neue Format für DNA-Arrays genutzt und weiterentwickelt, indem die spezifischen Sonden auf bzw. in dem Träger flexibel mittels in situ Synthese hergestellt werden, so dass ein Informationsfluß möglich wird. Jede neue Synthese des Arrays kann die Ergebnisse eines vorangegangenen Experimentes berücksichtigen. Durch geeignete Wahl von Sonden in Bezug auf ihre Länge, Sequenz und Verteilung auf dem Reaktionsträger und eine Rückkopplung des Systems mit integrierter Signalauswertung wird ein effizientes Prozessieren von genetischer Information möglich.

Durch die räumliche und zeitliche Kopplung von Herstellung und Auswertung (Analyse) der Arrays in einem Gerät, kann der Prozess und die Verwendung von Informationszyklen leicht automatisiert werden. Der Anwender legt in diesem Fall die Kriterien für die Auswahl (Selektionskriterien) fest.

Das erfindungsgemäße Verfahren ist grundsätzlich zur Bestimmung beliebiger Analyten geeignet, wie sie in Probenmaterial, insbesondere Proben biologischen Ursprungs vorkommen können. Besonders bevorzugt erfolgt eine Bestimmung von Nukleinsäuren-Analyten. Es können jedoch auch Proteine, Peptide, Glykoproteine, Arzneimittel, Drogen, metabolische Zwischenprodukte etc. bestimmt werden.

In einer bevorzugten Ausführungsform werden als Rezeptoren Polymersonden, insbesondere Nukleinsäuren oder deren Analoga, z.B. peptidische Nukleinsäuren (PNA) oder "locked nucleic acids" (LNA), verwendet. Es ist aber auch die Anwendung anderer Arten von Rezeptoren denkbar, oder eine Kombination mehrerer Arten von Rezeptoren, z.B. Peptide, Proteine, Saccharide, Lipide oder andere organische oder inorganische Verbindungen, die entsprechend in einem Array angeordnet werden können.

Die Bindung der Analyten an Rezeptoren an den jeweiligen Teilbereichen auf der Rezeptoroberfläche wird vorzugsweise über Markierungsgruppen nach-gewiesen. Die Markierungsgruppen können dabei direkt oder indirekt, z.B. über lösliche analytspezifische Rezeptoren, an den Analyten gebunden werden. Vorzugsweise werden Markierungsgruppen verwendet, die optisch detektierbar sind, z.B. durch Fluoreszenz, Lichtbrechung, Lumineszenz oder Absorption. Bevorzugte Beispiele für Markierungsgruppen sind fluoreszierende Gruppen oder optisch nachweisbare Metallpartikel, z.B. Goldpartikel.

Durch die sofortige Auswertung und anschließende Nutzung der gesammelten Daten wird das unten beschriebene Verfahren zu einem Lernprozess, mit dessen Hilfe es unter anderem möglich ist, z. B. in kurzer Zeit alle 25 Nukleotide langen Nukleinsäuren (25-mere) in einer vorgegebenen Sequenz zu bestimmen, ohne sie in ihrer Vielfalt (4²⁵ = 1.125899907 x 10¹⁵) synthetisieren zu müssen.

Dies kann in der bevorzugten Ausführungsform genutzt werden, um in einer unbekannten Sequenz oder einem Gemisch unbekannter Sequenzen eine Anzahl von Teilsequenzen mit geringer oder keiner Redundanz zu identifizieren, so dass schließlich in einer Art Filter die Teilmenge der tatsächlich vorhandenen Sequenzen von der Menge der theoretisch in einer Nukleinsäure möglichen Teilsequenzen abgetrennt wird.

Für die Expressionsanalyse ist auch die schnelle bzw. ökonomische und automatisierbare Auswahl eines ausgewählten Satzes an Polymersonden, der einer Subpopulation an Genen entspricht, aus einem gesamten Genom, das ggf. bereits als Sequenzinformation in einer Datenbank abgelegt ist, möglich.

Eine weitere wichtige Anwendung ist die empirisch gestützte Auswahl von Polymersonden-Sätzen mit bestimmten Eigenschaften. Diese Eigenschaften können im Fall von Nukleinsäuren-Sonden z.B. Bindeverhalten, Schmelzpunkt, Zugänglichkeit zu Zielmolekülen (targets) oder andere Eigenschaften sein, nach denen sich gezielt selektionieren lässt.

In einer anderen Ausführungsform wird nicht die Zusammensetzung der Rezeptoren, sondern die Geometrie der Arrays während des Verfahrens variiert. Dies kann z.B. die Größe des Messfeldes sein, auf dem die Polymersonden synthetisiert werden (Syntheseplätze). Auch hier kann nach bestimmten Kriterien anhand des korrespondierenden Signals eine Optimierung erfolgen.

### 4. Ausführliche Beschreibung der Erfindung

### 4.1 Zahlenverhältnisse

In jeder, aus *m* Nukleotiden bestehenden Sequenz können maximal *m-n*+1 Teilsequenzen der Länge *n* auftreten. Dies bedeutet, dass für jede Gesamtsequenzlänge *m* eine spezifische Sequenzlänge *n* existiert, für die die Anzahl aller möglichen *n*-mere (4ⁿ) die Anzahl *m-n* + 1 der in der Gesamtsequenz möglichen Teilsequenzen der Länge *n* überschreitet.

Im *E.coli* Genom z. B., das aus ca. 4,6 x 10⁶ Nukleotiden besteht, können somit maximal ca. 4,6 x 10⁶ Sequenzabschnitte einer beliebigen Länge *n* auftreten. Wählt man *n* = 12, so ist die Anzahl aller 12-mere mit 4¹² = 16777216 deutlich größer als die maximale Anzahl der im *E. coli* Genom auftretenden 12-mere. Es können also auf keinen Fall alle 12-mere und somit auch niemals alle längeren (*n* + 1)-, (*n* + 2)-mere, usw. in diesem Genom auftreten.

**Tabelle 1:**

| Wahrscheinlichkeiten (in %) für das Auftreten eines n-meren in einer Sequenz der Länge *m* | | | | | | | | | |
|---|---|---|---|---|---|---|---|---|---|
| **n/m** | **500** | **1000** | **10000** | **50000** | **100000** | **500000** | **1000000** | **5000000** | **10000000** |
| **1** | 100.0000 | 100.0000 | 100,0000 | 100,0000 | 100.0000 | 100.0000 | 100.0000 | 100.0000 | 100.0000 |
| **2** | 31.1875 | 62.4375 | 100.0000 | 100.0000 | 100.0000 | 100.0000 | 100.0000 | 100,0000 | 100.0000 |
| **3** | 7.7813 | 15.5938 | 100.0000 | 100.0000 | 100,0000 | 100.0000 | 100.0000 | 100.0000 | 100.0000 |
| **4** | 1.9414 | 3.8945 | 39.0508 | 100,0000 | 100.0000 | 100.0000 | 100,0000 | 100,0000 | 100,0000 |
| **5** | 0.4844 | 0.9727 | 9.7617 | 48.8242 | 97.6523 | 100.0000 | 100.0000 | 100,0000 | 100.0000 |
| **6** | 0.1208 | 0.2429 | 2.4402 | 12.2058 | 24,4128 | 100,0000 | 100,0000 | 100.0000 | 100.0000 |
| **7** | 0.0302 | 0.0607 | 0.6100 | 3.0514 | 6.1031 | 30.5172 | 61.0348 | 100.0000 | 100.0000 |
| **8** | 0.0075 | 0.0152 | 0.1525 | 0.7628 | 1.5258 | 7.6293 | 15.2587 | 76.2938 | 100.0000 |
| **9** | 0.0019 | 0.0038 | 0.0381 | 0.1907 | 0.3814 | 1.9073 | 3.8147 | 19.0735 | 38.1469 |
| **10** | 0.0005 | 0.0009 | 0.0095 | 0.0477 | 0.0954 | 0.4768 | 0.9537 | 4.7684 | 9.5367 |
| **11** | 0.0001 | 0.0002 | 0.0024 | 0.0119 | 0.0238 | 0.1192 | 0.2384 | 1.1921 | 2.3842 |
| **12** | 0.0000 | 0.0001 | 0,0000 | 0,0030 | 0.0060 | 0.0298 | 0.0596 | 0.2980 | 0.5960 |
| **13** | 0.0000 | 0.0000 | 0.0000 | 0.0007 | 0.0015 | 0.0075 | 0.0149 | 0.0745 | 0.1490 |
| **14** | 0.0000 | 0,0000 | 0.0000 | 0.0002 | 0.0004 | 0.0019 | 0.0037 | 0.0186 | 0.0373 |
| **15** | 0.0000 | 0.0000 | 0.0000 | 0.0000 | 0.0001 | 0.0005 | 0.0009 | 0.0047 | 0.0093 |
| **16** | 0.0000 | 0.0000 | 0.0000 | 0.0000 | 0.0000 | 0.0001 | 0.0002 | 0.0012 | 0.0023 |
| **17** | 0.0000 | 0.0000 | 0.0000 | 0.0000 | 0.0000 | 0.0000 | 0.0001 | 0.0003 | 0.0006 |
| **18** | 0.0000 | 0.0000 | 0.0000 | 0.0000 | 0.0000 | 0.0000 | 0.0000 | 0.0001 | 0.0001 |
| **19** | 0.0000 | 0.0000 | 0.0000 | 0.0000 | 0.0000 | 0.0000 | 0.0000 | 0.0000 | 0.0000 |

Der oben beschriebene Sachverhalt wird in Tabelle 1 anschaulich dargestellt. Für ein beliebiges, aber fest gewähltes n-mer wird jeweils die Wahrscheinlichkeit berechnet, mit der es in einer Sequenz der Länge m auftritt, wenn man zur Vereinfachung eine Gleichverteilung aller n-mere voraussetzt. Die Wahrscheinlichkeit wird dabei bestimmt durch die Länge m der Sequenz, der Länge n der beobachteten Teilsequenz und der Anzahl aller möglichen Sequenzen der Länge n.

Es ist deutlich zu erkennen, dass die Werte für die Wahrscheinlichkeit sehr klein werden, sobald die Länge n der beobachteten Teilsequenz so groß wird, dass nicht mehr alle *n*-mere in der Sequenz der Länge *m* auftreten können. Dieses Verhältnis zwischen der Sequenzabschnittslänge *n*, der Sequenzlänge *m* und der in der Sequenz der Länge *m* enthaltenen maximalen Anzahl von Teilsequenzen der Länge *n* wird in Tabelle 2 dargestellt. In jeder Sequenz, die kürzer ist als der für *m* angegebene Wert, kann jeweils nur ein Teil aller möglichen Abschnitte der angegebenen Länge *n* vorkommen.

**Tabelle 2:**

| Verhältnis zwischen der Sequenzlänge *m* und der maximal möglichen Anzahl der in ihr enthaltenen unterschiedlichen *n*-mere | | |
|---|---|---|
| | Sequenzlänge | n-mere in der Sequenz |
| *n* | *m* | m - 4^n + 1 |
| 3 | 66 | 64 |
| 5 | 1028 | 1024 |
| 6 | 4101 | 4096 |
| 7 | 16390 | 16384 |
| 8 | 65543 | 65536 |
| 9 | 262152 | 262144 |
| 10 | 1048585 | 1048576 |
| 12 | 16777227 | 16777216 |
| 13 | 67108876 | 67108864 |
| 14 | 268435469 | 268435456 |
| 15 | 1073741838 | 1073741824 |
| 16 | 4294967311 | 4294967296 |
| 17 | 17179869200 | 17179869184 |
| 18 | 68719476753 | 68719476736 |
| 19 | 2,74878E+11 | 2,74878E+11 |
| 20 | 1,09951E+12 | 1,09951E+12 |
| 25 | 1,1259E+15 | 1,1259E+15 |

Für ein Array, auf dem alle Sonden der Länge *s* synthetisiert werden, bedeuten die obigen Überlegungen zum Beispiel, dass nach einer Hybridisierung mit der Ausgangssequenz bei geeignet gewählten Synthese- und Hybridisierungsbedingungen nie alle Sonden ein Signal liefern können. Bei einer geschickten Wahl der Sondenlänge s kann eine Obergrenze für die Anzahl der signalgebenden Sonden vorgeben werden, diese wird bestimmt durch s ≥ *m* + 1-SP, wobei *SP* die Anzahl der signalgebenden Sonden ist. Eine solche Obergrenze kann z.B. bei der Sequenzierung zur Bestimmung der Start-Sondenlänge von Bedeutung sein.

### 4.2 Dynamischer Arrayaufbau

### 4.2.1 Verfahren

Wie oben beschrieben, kann in jeder Sequenz nur ein Bruchteil der möglichen Nukleotidkombinationen einer Länge n genutzt werden, daher ist es sinnvoll, auch nur eine Auswahl dieser Kombinationen auf den Arrays zu synthetisieren, um die gewünschte Sequenz zu untersuchen.

Die Länge s der Startsonden, d.h. der Sonden auf dem ersten Array, kann nach unterschiedlichen, sich aus der Anwendung ergebenden Kriterien gewählt werden. Für das oben erwähnte Verfahren kann dies z. B. die maximal gewünschte Anzahl der signalgebenden Sonden sein. Sollen auf dem ersten Array alle möglichen Kombinationen einer gewissen Länge s synthetisiert werden, so ist z. B. die Größe des vorhandenen Arrays ein Kriterium zur Bestimmung der Sondenlänge, da die benötigte Stellptatzanzahl (4^{s}) die Anzahl der vorhandenen Stellplätze nicht überschreiten darf.

Für andere Anwendungen ist es u. a. denkbar, dass nur Sonden mit gleichen Eigenschaften, z. B. mit der gleichen Start- oder Endsequenz von Interesse sind, dies reduziert wiederum die Anzahl der möglichen Sonden.

Auf dem im ersten Bestimmungszyklus eingesetzten Array werden nun alle Sonden der gewählten Länge und Eigenschaften synthetisiert, gegen sie wird die zu untersuchende Sequenz hybridisiert. Wie oben beschrieben ist es unwahrscheinlich, dass nach der Hybridisierung von allen Stellplätzen Signale ausgehen, da bei geschickter Wahl der Sondenlänge nicht alle Sondensequenzen in der Ausgangssequenz vorkommen können. Zudem treten einige Sondensequenzen häufiger in der Ausgangssequenz auf, was zu Mehrfachbindungen an einzelne Stellplätze führt und somit die Anzahl der Signale reduziert.

Alle für die jeweilige Anwendung relevanten Stellplätze werden auf einem neuen Array variiert. Dies kann auf verschiedenen Arten geschehen.

### 4.2.2 Variation durch Verlängerung der Sonden / iterativer Sondenaufbau

Eine Möglichkeit, die Sonden auf einem neuen Array zu variieren ist es, sie in ihrer Länge zu verändern, d. h., sie durch Verlängerung um ein oder mehrere Nukleotide spezifischer zu machen. Dazu werden alle Sonden, die auf dem Vorgängerarray ein Signal erzeugt haben, auf einem neuen Array synthetisiert und jeweils um alle für die untersuchte Sequenzart relevanten Nukleotidbausteine verlängert. Für eine Untersuchung von DNA/RNA-Sequenzen bedeutet dies z. B. eine Verlängerung jeder Sonde um die vier Nukleotide Adenin, Thymin, Guanin und Cytosin. In diesem Fall werden für einen Stellplatz auf dem Vorgängerarray vier Stellplätze auf dem neuen Array benötigt, für jedes der vier Nukleotide einer, siehe Tabelle 3. In allen anderen Fällen ergeben sich auf dem neuen Array so viele Stellplätze pro Stellplatz auf dem Vorgängerarray, wie es Bausteine gibt, um die die Sonden erweitert werden können.

**Tabelle 3:**

| Beispiel für die Sondenverlängerung bei DNA-/RNA-Sequenzen | | | | |
|---|---|---|---|---|
| **Alter Stellplatz** | **Neue Stellplätze** | | | |
| | A | C | G | T |
| N | N | N | N | N |
| N | N | N | N | N |
| N | N | N | N | N |
| N | N | N | N | N |
| N | N | N | N | N |
| N | N | N | N | N |
| N | N | N | N | N |
| N | N | N | N | N |
| N | N | N | N | N |
| N | N | N | N | N |

Gegen die neu synthetisierten Sonden des Folgearrays wird die Ausgangssequenz hybridisiert; auch nach diesem Vorgang werden nicht alle Sonden ein Signal abgeben. Die relevanten Sonden werden auf einem neuen Array aufgebaut und weiter verlängert, die neue Stellplatzanzahl ist somit immer das Vierfache der Signalanzahl auf dem Vorgängerarray. Dieses Vorgehen wird so lange wiederholt bis eine vorher festgelegte maximale Sondenlänge erreicht wird.

### 4.2.3 Filterwirkung des iterativen Aufbaus

Der hier beschriebenen iterativen Aufbau der für die Untersuchung der Ausgangssequenz relevanten Sonden wirkt wie ein Filter, der unabhängig von der Sondenlänge die Sonden aussortiert, die kein Signal geliefert haben. Auf jedem neuen Array werden dann so viele Sonden zur Verfügung gestellt, wie es Möglichkeiten gibt, eine erfolgreiche Sonde zu verlängern. Nach dem Überschreiten einer von der Länge und Art der Ausgangssequenz abhängigen spezifischen Sondenlänge wird sich die Anzahl der Signale auf den folgenden Arrays nicht weiter vergrößern, die Stellplatzanzahl bleibt also annähernd konstant. Das Verfahren ermöglicht es somit für die jeweilige Anwendung wichtige, sehr spezifische Sonden zu selektieren und nur diese zu synthetisieren. Jede Probensequenz kann also mit der Vielfalt der Oligonukleotide von spezifischer Länge verglichen werden, ohne alle möglichen Kombinationen dieser Länge erzeugen zu müssen, es besteht somit keine Einschränkung in der Kombinationsvielfalt bei der Untersuchung der Ausgangssequenzen.

Die Kriterien für eine erfolgreiche Sonde können dabei als Parameter variiert bzw. abhängig von den Zielen der Optimierung festgelegt werden. Eine solche Festlegung könnte auch die Auswahl eines Anteils der Sonden sein, die ein bestimmtes Signal zeigen, also z.B. eine gewisse festgelegte Schwelle übersteigen. Diese Schwelle kann wiederum abhängig vom Gesamtsignal gelegt werden, so dass z.B. die 25 % Polymersonden mit dem höchsten Signal als erfolgreich eingestuft werden. Andere Kriterien wären z.B. die Kinetik der Bindungsreaktion oder die Spezifität der Bindung.

Eine Sequenz von 50.000 Nukleotiden enthält, wie in 4.1 beschrieben, maximal 50.000 verschiedene Teilsequenzen einer Länge *n*. Wählt man in diesem Fall n = 8, so gibt es mehr 8-mere (4⁸ = 65536) als in dieser Sequenz vorkommen können. Werden auf dem ersten Array nun alle 8-mere synthetisiert, so können nach der Hybridisierung nicht von allen Sonden Signale ausgehen. Die relevanten Sonden werden nun auf dem folgenden Array verlängert, die benötigte Stellplatzanzahl des Folgearray ist somit 4 x Signalanzahl, auf jeden Fall aber kleiner als 4 x 4⁸ = 262144. In keinem Fall wird die auf den Folgearrays benötigte Stellplatzanzahl diesen Wert übersteigen.

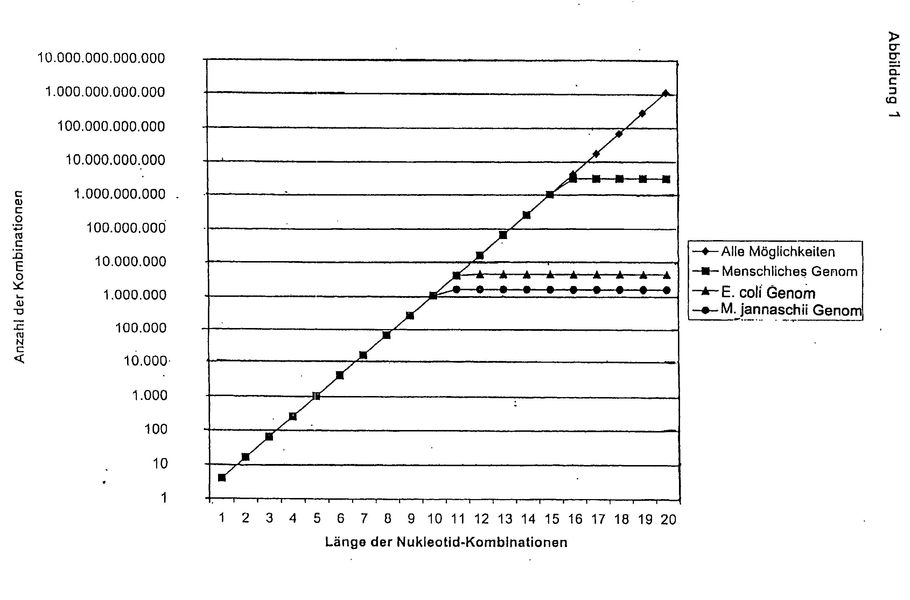

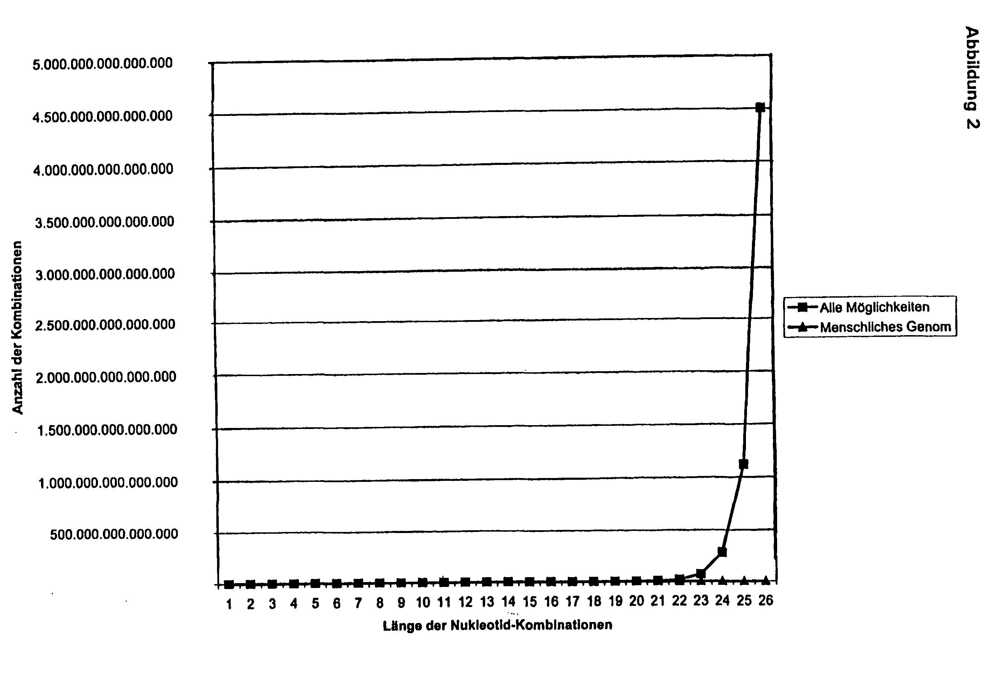

Die Abbildungen 1 und 2 zeigen das Verhältnis zwischen der Anzahl aller möglichen Sequenzen der Länge n und der Anzahl der potentiell möglichen unterschiedlichen Teilsequenzen, die im menschlichen Genom, im Genom von *E. coli* und im *M. jannaschii* Genom vorkommen können. Die Anzahl aller möglichen Kombinationen (4ⁿ) nimmt exponentiell zu, während die in den Genomen mögliche Anzahl der Teilsequenzen mit dem Erreichen einer spezifischen Länge nicht weiter zunimmt. Die Natur nutzt nur wenige der vorhandenen Möglichkeiten, diese können mit einem lernenden System, dem das hier beschriebene Verfahren zugrunde liegt, detektiert werden.

Sind in einzelnen Iterationsschritten Signale nicht eindeutig auswertbar, so können diese Sonden als relevante Sonden betrachtet und auf den neuen Arrays weiter aufgebaut werden. Mit zunehmender Länge der Sonden wird die Hybridisierung spezifischer und die Aussage erwartungsgemäß eindeutiger.

### 4.2.4 Optimierung und Verifikation der Ergebnisse durch Variation der Sonden

Neben der oben beschrieben Verlängerung können Sonden auch auf andere Arten von einem Array zum nächsten variiert werden. So kann - bei polymeren Sonden - auch eine Variation innerhalb der Sondensequenz durch Substitution einzelner Bausteine, z.B. Nukleotide, durch andere Bausteine erfolgen. Weiterhin können die Position oder/und die Dichte von Rezeptoren auf der Trägerfläche variiert werden. Auch eine Variation in der Art der Kopplung von Rezeptoren auf der Trägeroberfläche, z.B. hinsichtlich der hierzu verwendeten Linkermoleküle ist möglich. Darüber hinaus können die Bindungsbedingungen zwischen Analyt und Rezeptor in aufeinanderfolgenden Bestimmungszyklen variiert werden, wobei z.B. bei Nukleinsäure-Analyten eine Variation der Hybridisierungsbedingungen (z.B. Salzgehalt, Temperatur, Fluidbewegung oder andere Parameter) variiert werden können. Schließlich können auch die Synthesebedingungen beim Aufbau des Rezep-tors, z.B. bei der Kopplung vollständiger Rezeptoren und insbesondere beim Aufbau des Rezeptors aus mehreren Synthon-Bausteinen variiert werden.

So kann zum Beispiel die Position des Stellplatzes oder die Dichte der Stellplätze einen Einfluß auf die Hybridisierungs - oder/und Synthesebedingungen haben, so dass das nach der Hybridisierung erzielte Ergebnis nicht eindeutig zuzuordnen ist. Durch die Wahl einer neuen Stellplatzposition oder eine veränderte Stellplatzdichte auf dem folgenden Array kann eventuell ein besseres positives Signal erzeugt werden, bzw. das Fehlen eines Signals bestätigt werden. Dies ermöglicht es unter anderem im Verlauf des Verfahrens Erfahrungen über die Hybridisierungs- und Synthesebedingungen der einzelnen Sonden zu sammeln. Die Ergebnisse können z. B. in einer Datenbank abgelegt werden, um bei einer ähnlichen Problemstellung wieder Anwendung zu finden. Mit Hilfe der erzeugten Daten kann das System für jede Problemstellung optimiert werden, so dass z. B. im Laufe der Zeit, bzw. in dafür ausgelegten Versuchen, Sonden selektiert werden können, mit denen sich die gleiche Problemstellung für unterschiedliches Probenmaterial lösen läßt.

Werden nur ausgewählte Sonden auf einem Array synthetisiert, so ist es möglich relevant erscheinende Sonden im nächsten Schritt nur in einzelnen Stellen der Sequenz zu verändern, also einzelne Nukleotide gegen andere auszutauschen. Welche Sonden für eine solche Modifikation in Frage kommen, muß für jeden Anwendungsfall getrennt festgelegt werden.

### 4.3 Ausführungsbeispiele

An zwei Beispielen soll verdeutlicht werden, wie das oben beschriebene Verfahren z.B. angewendet werden kann, um alle n-mere von spezifischer Länge einer Sequenz zu bestimmen, ohne die zu untersuchende Sequenz gegen alle existierenden n-mere vergleichen zu müssen.

Im ersten Beispiel wird das aus ca. 1,6 Millionen Nukleotiden bestehende *M.jannaschii* Genom untersucht. Mit Hilfe einer Simulation werden zunächst alle 9-mere dieses Genoms (zur Vereinfachung einzelsträngig) bestimmt. Von 262.144 möglichen Kombinationen von 9 Nukleotiden Länge treten in einem Strang des untersuchten Genoms 177.167 Kombinationen auf. Im nächsten Schritt werden alle relevanten Sonden verlängert; nach erneuter Hybridisierung gehen von 436.325 der 708.668 Stellplätze auf dem neuen Array Signale aus. In der Simulation wird dieses Vorgehen bis zu einer Länge von 13 Nukleotiden wiederholt. Nach der Hybridisierung im letzten Schritt gehen von 1.441.322 Stellplätzen Signale aus. Dies ist nur ein Bruchteil der insgesamt möglichen 67.108.864 Kombinationen von 13 Nukleotiden Länge.

Insgesamt können in einem Strang des *M.jannaschii* Genoms bis zu ca. 1,6 Millionen verschiedene Teilsequenzen einer Länge n auftreten. Das Verfahren nähert sich mit jedem Schritt dieser Obergrenze, kann sie aber nie überschreiten. Dies bedeutet unter anderem, dass in keinem Schritt mehr als 6,4 Millionen Stellplätze benötigt werden, was im Vergleich zur Vielfalt aller möglichen Kombinationen eine relativ geringe Zahl ist.

Im zweiten Beispiel wird ein 188.642 Nukleotide langes Gen des Menschen untersucht. Zur Vereinfachung wird auch in dieser Simulation ein Einzelstrang gewählt.

Im ersten Schritt werden alle möglichen Sonden von 6 Nukleotiden Länge (4096) auf einem Array synthetisiert. Die Wahrscheinlichkeit, mit der eine Sondensequenz mehr als einmal in der zu untersuchenden Sequenz auftritt liegt bei 100%, deshalb gehen nach der Hybridisierung von allen Stellplätzen Signale aus, die Sondenlänge wurde also zu kurz gewählt. Im nächsten Schritt müssen daher alle 7-mere synthetisiert werden, also 16.384 Stück. Nach der Hybridisierung gibt es 14.803 relevante Sonden, die auf einem neuen Array synthetisiert und verlängert werden. Dieses Vorgehen wird bis zu einer Sondenlänge von 20 Nukleotiden wiederholt. Nach der letzten Hybridisierung gehen von 180.362 Stellplätzen Signale aus. Im Laufe des Verfahrens nähert sich die Anzahl der relevanten Sonden der maximal möglichen Anzahl von ungefähr 188.600, wie im ersten Beispiel kann diese Zahl aber nicht überschritten werden.

Somit ermöglicht das erfindungsgemäße Verfahren die Bestimmung von Teilsequenzen mit spezifischer Länge, ohne alle Sequenzen dieser Länge erzeugen zu müssen.

### 4.4 Probenvorbereitung

Das erfindungsgemäße Verfahren kann sowohl mit einzelsträngiger RNA oder DNA (ssRNA bzw ssDNA) als auch mit doppelsträngigen Nukleinsäuren, z.B. dsRNA bzw. dsDNA durchgeführt werden. Die Nukleinsäuren werden dazu entsprechend dem Stand der Technik aus Viren, Bakterien, Pflanzen, Tieren oder dem Menschen isoliert oder können aus anderen Quellen stammen.

Einzelsträngige Nukleinsäuren werden in der Mehrzahl der Fälle ausgehend von dsDNA durch spezielle *in vitro* Verfahren erzeugt. Hierzu zählen z.B. asymmetrische PCR (erzeugt ssDNA), PCR mit derivatisierten Primern, die eine selektive Hydrolyse eines einzelnen Stranges im PCR-Produkt ermöglichen, oder die Transkription durch RNA-Polymerasen (erzeugt ssRNA). Als Matrizen können bei der Transkription neben nicht klonierter einzelsträngiger DNA vor allem auch in spezielle Vektoren (z.B. Plasmidvektoren mit einem Promotor; Plasmidvektoren mit zwei unterschiedlich orientierten Promotoren für eine bestimmte oder zwei unterschiedliche RNA-Polymerasen) klonierte dsDNA eingesetzt werden. Die in die Plasmide klonierte Insert-DNA oder die bei der PCR eingesetzte DNA-Matrize können zum einen aus Viren, Bakterien, Pflanzen, Tieren oder dem Menschen isoliert werden, zum anderen aber prinzipiell auch *in vitro* durch reverse Transkription, RNaseH-Behandlung und anschließende Amplifikation (z.B. durch PCR) aus ssRNA erzeugt werden. Als RNA-Matrizen sind hier neben rRNAs, tRNAs, mRNAs und snRNAs auch *in vitro* erzeugte Transkripte (entstanden z.B. durch Transkription mit SP6-, T3- oder T7-RNA-Polymerase) geeignet. Für den Fachmann sind noch weitere Methoden denkbar.

Doppelsträngige Nukleinsäuren können z.B. aus dsDNA gewonnen werden. Diese dsDNA kann zum einen als genomische, chromosomale DNA, als extrachromosomales Element (z.B. als Plasmid) oder als Bestandteil von Zellorganellen aus Viren, Bakterien, Tieren, Pflanzen oder dem Menschen isoliert werden, zum anderen aber prinzipiell auch in vitro durch reverse Transkription, RNaseH-Behandlung und anschließende Amplifikation (z.B. durch PCR) aus ssRNA erzeugt werden. Als RNA-Matrizen können hierbei neben rRNAs, tRNAs, mRNAs und snRNAs wiederum in vitro erzeugte Transkripte (entstanden z.B. durch Transkription mit SP3-, T3- oder T7-RNA-Polymerase) eingesetzt werden.

Die für das Verfahren vorgesehenen Nukleinsäuren werden vorzugsweise sequenzspezifisch oder/und sequenzunspezifisch fragmentiert (z.B. durch sequenz(un)spezifische Enzyme, Ultraschall oder Scherkräfte), wobei eine vorbestimmte, z.B. im Wesentlichen homogene Längenverteilung der Bruchstücke/Hydrolyseprodukte angestrebt wird. Wird die vorbestimmte Längenverteilung der Fragmente zunächst nicht erreicht, kann anschließend eine Längenfraktionierung z.B. durch gelelektrophoretische oder/und chromatographische Verfahren durchgeführt werden, um die gewünschte Längenverteilung zu erhalten. Es kann allerdings auch Anwendungen geben, bei denen eine definierte Fragmentierung durchgeführt wird, z.B. unter Verwendung sequenzspezifischer Enzyme oder Ribozyme.

Die entstehenden Fragmente werden vorzugsweise markiert, z.B. mit fluoreszierenden Agenzien, weitere Möglichkeiten sind der Einbau radioaktiver Isotope, lichtbrechende Partikel oder enzymatische Marker wie Peroxidase. Die Markierung erfolgt dabei bevorzugt an den Enden der Fragmente (terminale Markierung). 3'-terminale Markierungen können unter Verwendung geeigneter Synthone z.B. mit der terminalen Transferase oder der T4 RNA-Ligase durchgeführt werden. Werden für die Fragmentierung *in vitro* erzeugte RNA-Transkripte eingesetzt, kann die Markierung auch vor der Fragmentierung durch bei der Transkription eingesetzte markierte Nukleotide erfolgen (interne Markierung). Weitere Verfahren wie *Nick Translation* sind dem Fachmann bekannt.

Die markierten, fragmentierten Nukleinsäuren können dann in einer geeigneten Hybridisierungslösung gegen das Oligonukleotid-Array hybridisiert werden.

### 5. Anwendungen

Das erfindungsgemäße Verfahren kann in einer Ausführungsform für die Analyse von differentieller Expression genutzt werden. Dazu werden zwei Proben A und B aus unterschiedlichen Zellen gewonnen, die miteinander verglichen werden sollen. Dabei könnte A eine normale Zelle und B eine Krebszelle sein. Beliebige andere Unterschiede sind möglich.

Die Proben werden nun mit Hilfe dynamischer lernender Arrays charakterisiert und diejenigen Sonden als negativ eingestuft, d.h. haben definitionsgemäß kein Signal ergeben, die in beiden Proben ausreichend ähnlich oder gleich repräsentiert sind. Weiterverfolgt werden hingegen solche Sonden, bei denen nur bei einer der beiden Proben ein Signal zu sehen war. So werden zunehmend spezifische Sonden selektiert, die nur in einer der beiden Proben komplementäre Sequenzen vorfinden. Bei einer Länge von 25 - 30 Nukleotiden ist für den Menschen eine solche Sonde selbst unter Berücksichtigung aller vorhandenen Gene (die nie alle gleichzeitig exprimiert werden), die nur 1-10% der genomischen DNA ausmachen, hochspezifisch. Damit werden die selektierten Sonden zu Markern für differentiell exprimierte Gene oder zumindest Spleissvarianten. Gleichzeitig kann man aber auch ein Korrelat für EST's darin sehen, da bei entsprechender Sondenlänge 30-40 Basenpaare der Sequenz bestimmt sein könnten. Wenn für das humane Genom 30mere als differentielle Marker bestimmt sind, dann reicht diese Länge der Sonde mit hoher Wahrscheinlichkeit aus, um das entsprechende mRNA Molekül eindeutig identifizieren zu können.

Die Sonden können in einem weiteren Arbeitsschritt als Fängersonden (capture probes) für die spezifische Isolation der entsprechenden mRNA Population genutzt werden. Auf diese Weise kann Material gewonnen werden, das für weitere Untersuchungen wie eine Sequenzierung oder Klonierung zur Verfügung steht.

Damit lassen sich zum einen Klone aus einer Bibliothek fischen, z.B. mit etablierten Verfahren wie Blots und Filtern aus Bibliotheken in Bakterien, Hefen oder anderen geeigneten Zellen. Andererseits lassen sich diese Oligo-EST's auch nutzen, um von hier aus mit bekannten Verfahren wie *Primer Walking* oder mit anderen Verfahren weitere Teile der Sequenz zu entschlüsseln.

In einer Variante kann das erfindungsgemäße Verfahren genutzt werden, um geeignete Fängersonden (capture probes) in einem entsprechenden lernenden Verfahren zu optimieren. Dies kann z.B. im Hinblick auf ihre Spezifität oder/und ihre Zugänglichkeit für die Zielmoleküle erfolgen.

Auch andere Oligonukleotide können im erfindungsgemäßen Verfahren auf Eigenschaften wie eine bestimmte Funktion, die Spezifität der Bindung oder/und Zugänglichkeit zum Zielmolekül hin optimiert werden. Solche Oligonukleotide sind z.B. Antisense-Moleküle und Ribozyme.

In einer weiteren Variante des Verfahrens werden Phagenbibliotheken oder ähnliche biologisch funktionelle Bibliotheken mit Hilfe des erfindungsgemäßen Verfahrens auf bestimmte Optimierungsziele hin selektioniert. Der Vorteil eines solchen Einsatzes ist die parallele Optimierung der Sonden auf der festen Phase und die Selektion einer Population aus der Bibliothek. So können Optimierungsprozesse beschleunigt werden.

In noch einer weiteren Variante können auf weiteren Arrays die differentiellen Sonden ohne weitere Charakterisierung verwendet werden, um damit weitere Proben zu untersuchen, z.B. Zellen, die einem ähnlichen Krankheitsbild zugeordnet werden. So kann ohne weitere Arbeit wie Klonieren, funktionellen Studien etc. ein Zusammenhang hergestellt oder eine für diagnostische Zwecke sinnvoll erscheinende Kombination von Sonden etabliert werden. Damit wird ein großer Teil eines Screening-Ansatzes mit hohem Durchsatz und relativ geringem Aufwand an molekularbiologischen und biochemischen Experimenten möglich, und erst interessante Sonden bzw Oligo-EST's werden in weiterführende Arbeiten übernommen.

Ein Aspekt der beschriebenen Anwendungen ist, dass weitgehend undefiniertes Material ohne Vorkenntnisse über die Sequenz der darin enthaltenen Nukleinsäure effizient nach differentiell exprimierten oder differentiell repräsentierten Sonden und damit ggf Genen oder Spleissprodukten durchsucht werden kann. Man benötigt nur eine Vergleichsprobe, gegen die die Differenzierung vorgenommen wird.

Ein weiterer wesentlicher Vorteil der beschreibenen Vorgehensweise liegt darin, dass der Selektionsprozess die Optimierung der Sonden auf stabile Hybridisierung, Zugänglichkeit der Zielsequenz und Deutlichkeit des Signales hin bereits beinnhaltet. Es werden quasi systemimmanent die für ein deutliches Signal am besten geeigneten Sonden ausgewählt, die dann zudem hochspezifisch sind.

In einer weiteren Ausführungsform werden die beschriebenen Mechanismen eingesetzt, um genomische DNA in zwei Proben zu vergleichen. So können z.B. chromosomale Aberrationen wie Deletionen etc. identifiziert werden.

In einer anderen Ausführungsform werden genomische DNA Populationen verglichen, um sogenannten Einzelnukleotid-Polymorphismen (SNP) zu identifizieren. Dazu mag es zweckmäßig sein, die DNA aus zwei oder mehr Probenquellen zu vergleichen. Für den Prozess des Vergleiches kann es auch von Interesse sein, bei bekannten SNP's den Gehalt von zwei oder mehr Genomen auf diese SNP's hin zu untersuchen, um in einem automatisierten Verfahren die unterschiedlichen SNP's zu finden.

Ein weiterer Aspekt der Erfindung ist die Möglichkeit zur Optimierung der physiko-chemischen Eigenschaften der Polymersonden. Dazu zählt zum Beispiel die Länge des Linker-Moleküls, das einen Rezeptor mit der festen Phase verbindet, seine Ladung oder andere Charakteristika des Linkers, die das Bindungsereignis an den Rezeptor beeinflussen. Auch Effekte durch Wechselwirkung von Rezeptoren auf benachbarten Feldern und die unterschiedliche Zugänglichkeit von Probenmaterial für die Rezeptoren können systematisch optimiert werden.

Als weitere physiko-chemische Eigenschaft könnte die Schmelztemperatur oder Duplexstabilität bei bestimmten Rahmenbedingungen wie z.B. dem Salzgehalt im Puffer optimiert werden.

Dieser Prozess ist dann prinzipiell geeignet, um Bibliotheken von Polymersonden mit bestimmten Eigenschaften zu entwickeln. Ein Beispiel wäre eine Bibliothek von 25-30 Basen langen Oligosonden, die bei einer vorbestimmten Temperatur, z.B. 35°C, ihren Schmelzpunkthaben (definiert als Tm). Eine solche empirisch entwickelte Bibliothek ist von sehr großem Wert für die Auswahl von entsprechenden Oligosonden für unterschiedliche Anwendungen, insbesondere für die Anwendung als Sonden auf einem Array. Die Bibliothek kann bei der Entwicklung eines neuen Arrays für eine bestimmte Fragestellung, z.B. den Nachweis der Expression einer kleinen Auswahl an Genen aus einem größeren Genom wie dem Humangenom verwendet werden, um schnell geeignete und empirisch validierte Sonden in den Auswahlprozess einzubeziehen.

Andere Bibliotheken können nach einer bestimmten Länge ausgewählt sein. Aus verschiedenen Bibliotheken können Sonden wiederum gemischt werden. Der Auswahlprozess kann dabei so ablaufen, dass, ausgehend von einer bestimmten Anzahl an Stellplätzen, die maximal mögliche Varianz an Oligomeren aufgebaut wird. Dies wären im Fall von 64.000 Stellplätzen in etwa alle 8mere. Dieser Array wird mit einer Mischung aller 8mere als Probe hybridisiert und die 25 % Sonden mit dem stärksten Signal selektiert. Diese erfolgreichen Sonden werden nun in einem neuen Array zu 9meren verlängert. So kann eine Bibliothek von Oligomeren der Länge n entstehen, nach n-Ausgangslänge Informationszyklen, die aus b = (Anzahl der Stellplätze) möglichen Mitgliedern besteht. Damit werden viele dem Fachmann bekannten Probleme mit der rein theoretischen Vorhersage von geeigneten Oligosonden durch ein empirisches Verfahren gelöst. Für eine große Zahl b kann eine Generation an Oligosonden auch parallel oder nacheinander in verschiedenen Reaktionsträgern aufgebaut werden. So könnte mit ca. 1 Million Stellplätzen mit n = 10 begonnen werden.

Darüber hinaus eignet sich das Verfahren auch zur Optimierung des Herstellprozesses oder zu vergleichenden Untersuchungen zur Synthesequalität.

Ein anderer Aspekt der Erfindung ist die Konzeption von Diagnose-Systemen, z.B. von individualisierten oder/und mehrstufigen Diagnose-Systemen, die eine analytische Anwort ebenfalls in lernenden Zyklen erarbeiten und z.B. in 2 oder mehr Zyklen das Probengut untersuchen. Dabei könnte die erste Runde bzw der erste Array einer Art "Pre-Scan" in Analogie zu einem Bildscanner dienen, während darauf folgend dann an den als relevant erkannten Stellen weiter in die Tiefe gesucht wird. So könnte in einer konkreten Anwendung erst der Expressionszustand erfaßt werden, um dann bei denjenigen Genen, die eine Abweichung zeigen, im Detail die Sequenz zu erfassen oder bestimmte bekannte Aberationen, Mutationen oder SNP's zu bestimmen. Dabei kann die Probe z.B. mit einem Standard verglichen werden, und aus diesem Vergleich kann dann ggf. die Art der weiteren Analyse folgen, z.B. Auswahl von diagnostischen Kombinationen an Polymersonden auf dem nächsten Array. In einer weiterführenden Anwendung ist es denkbar, aus diesem Ansatz dann "dynamische" Tests zu entwickeln, bei denen es darum geht, das Probengut durch mehrere Schleifen der Veränderung oder Optimierung des Arrays zu schicken, bis z.B. die diagnostische Antwort eine statistische Schwelle (Signifikanz etc.) überschreitet.

Insgesamt eignet sich ein flexibles, auf Basis von Selektionsvorgängen evolutiv arbeitendes System wie das erfindungsgemäße Verfahren besser als starre Arrays, um der Plastizität des Lebens und seiner Erscheinungsformen eine Plastizität der analytischen Werkzeuge und Fragestellungen entgegenzuhalten, um damit auch angesichts der Masse an Information in biologischen Systemen mit begrenztem Aufwand zu sinnvollen Aussagen zu kommen.

## Patentansprüche

1. Verfahren zur Bestimmung von Analyten in einer Probe umfassend die Schritte:
(a) Durchführen eines ersten Bestimmungszyklus umfassend:
(i) Bereitstellen eines geschlossenen Trägers mit einer Oberfläche auf einer innen liegenden Struktur, die an einer Vielzahl von vorbestimmten Bereichen immobilisierte Rezeptoren enthält, wobei die Rezeptoren in einzelnen Bereichen auf dem Reaktionsträger jeweils eine unterschiedliche Analytspezifität aufweisen,
(ii) Inkontaktbringen der Probe, die zu bestimmende Analyten enthält, mit dem Träger unter Bedingungen, bei denen eine Bindung zwischen den zu bestimmenden Analyten und dafür spezifischen Rezeptoren auf dem Träger erfolgen kann, und
(iii) Identifizieren der vorbestimmten Bereiche auf dem Träger, an denen eine Bindung in Schritt (ii) erfolgt ist,
(b) Durchführen eines nachfolgenden Bestimmungszyklus umfassend:
(i) Bereitstellen eines weiteren geschlossenen Trägers mit einer Oberfläche auf einer innen liegenden Struktur, die an einer Vielzahl von vorbestimmten Bereichen immobilisierte Rezeptoren enthält, wobei die Rezeptoren in einzelnen Bereichen auf dem Reaktionsträger jeweils eine unterschiedliche Analytspezifität aufweisen, wobei fürden weiteren Träger Rezeptoren ausgewählt werden, bei denen in einem vorhergehenden Zyklus ein vorbestimmtes charakteristisches Signal, das für die Bindung des Analyten an einen spezifischen Rezeptor charakteristisch ist, beobachtet worden ist, und wobei die ausgewählten Rezeptoren oder/und die Bedingungen der Rezeptor-Analyt-Bindung gegenüber einem vorhergehenden Bestimmungszyklus verändert werden,
(ii) Wiederholen von Schritt (a) (ii) mit dem weiteren Träger und
(iii) Wiederholen von Schritt (a) (iii) mit dem weiteren Träger und
(c) gegebenenfalls Durchführen von einem oder mehreren weiteren nachfolgenden Bestimmungszyklen jeweils mit Auswahl und Veränderung der Rezeptoren gemäß Schritt (b) (i), bis eine ausreichende Information über die zu bestimmenden Analyten vorliegt oder/und bis das Signal nach erfolgtem Bindungsereignis einem vorbestimmten Kriterium entspricht, wobei die Durchführung von Bestimmungszyklen die integrierte Herstellung und Auswertung eines Trägers in einem Gerät umfasst ist.

2. Verfahren nach Anspruch 1,
**dadurch gekennzeichnet,**
**dass** man Nukleinsäure-Analyten bestimmt.

3. Verfahren nach Anspruch 2,
**dadurch gekennzeichnet,**
**dass** die Nukleinsäure-Analyten ausgewählt werden aus doppelsträngiger DNA, einzelsträngiger DNA und RNA.

4. Verfahren nach Anspruch 2 oder 3,
**dadurch gekennzeichnet,**
**dass** die Nukleinsäure-Analyten vor dem Inkontaktbringen mit dem Träger sequenzspezifisch oder/und sequenzunspezifisch fragmentiert werden.

5. Verfahren nach Anspruch 4,
**dadurch gekennzeichnet,**
**dass** durch die Fragmentierung und gegebenenfalls eine nachfolgende Längenfraktionierung Nukleinsäurefragmente mit einer vorbestimmten Längenverteilung erzeugt werden.

6. Verfahren nach Anspruch 4 oder 5,
**dadurch gekennzeichnet,**
**dass** Nukleinsäurefragmente mit einer im Wesentlichen homogenen Längenverteilung erzeugt werden.

7. Verfahren nach einem der vorhergehenden Ansprüche,
**dadurch gekennzeichnet,**
**dass** die Analyten Markierungsgruppen tragen.

8. Verfahren nach Anspruch 7,
**dadurch gekennzeichnet,**
**dass** die Markierungsgruppen optisch detektierbar sind.

9. Verfahren nach Anspruch 7 oder 8,
**dadurch gekennzeichnet,**
**dass** Fluoreszenzmarkierungen oder/und Metallpartikelmarkierungen verwendet werden.

10. Verfahren nach einem der vorhergehenden Ansprüche,
**dadurch gekennzeichnet,**
**dass** die Rezeptoren aus polymeren Sonden ausgewählt werden.

11. Verfahren nach Anspruch 10,
**dadurch gekennzeichnet,**
**dass** die Veränderung der Rezeptoren eine Veränderung der Sondensequenz umfaßt.

12. Verfahren nach Anspruch 10 oder 11,
**dadurch gekennzeichnet,**
**dass** die Veränderung eine Verlängerung der Sondensequenz umfaßt.

13. Verfahren nach Anspruch 10 oder 11,
**dadurch gekennzeichnet,**
**dass** die Veränderung eine Variation in der Sondensequenz umfaßt.

14. Verfahren nach einem der vorhergehenden Ansprüche,
**dadurch gekennzeichnet,**
**dass** die Veränderung eine Variation der Position oder/und Dichte von Rezeptoren auf der Trägeroberfläche umfaßt.

15. Verfahren nach einem der vorhergehenden Ansprüche,
**dadurch gekennzeichnet,**
**dass** die Veränderung eine Variation in der Art der Kopplung von Rezeptoren auf der Trägeroberfläche umfaßt.

16. Verfahren nach Anspruch 15,
**dadurch gekennzeichnet,**
**dass** zur Kopplung der Rezeptoren verwendete Linkermoleküle variiert werden.

17. Verfahren nach einem der vorhergehenden Ansprüche,
**dadurch gekennzeichnet,**
**dass** die Veränderung eine Variation der Bindungsbedingungen zwischen Analyt und Rezeptor umfaßt.

18. Verfahren nach Anspruch 17,
**dadurch gekennzeichnet,**
**dass** bei Nukteinsäure-Analyten eine Variation der Hybridisierungsbedingungen erfolgt.

19. Verfahren nach einem der vorhergehenden Ansprüche,
**dadurch gekennzeichnet,**
**dass** die Veränderung eine Variation der Synthesebedingungen bei einem Aufbau des Rezeptors auf der Trägeroberfläche umfaßt.

20. Verfahren nach einem der Ansprüche 1 bis 14,
**gekennzeichnet dadurch,**
**dass** die Veränderung eine Variation der Stellplatzgeometrie, insbesondere der Stellplatzgröße, umfasst.

21. Verfahren nach einem der Ansprüche 1 bis 19,
**dadurch gekennzeichnet,**
**dass** die Veränderung eine empirische Auswahl bzw. gezielte Selektion einer Rezeptoren-Bibliothek umfasst.

22. Verwendung des Verfahrens nach einem der Ansprüche 1 bis 21 zur differentiellen Expressionsanalyse.

23. Verwendung des Verfahrens nach einem der Ansprüche 1 bis 21 zur differentiellen Genomanalyse.

24. Verwendung nach Anspruch 23 zur Identifizierung chromosomaler Polymorphismen oder Aberrationen.

25. Verwendung des Verfahrens nach einem der Ansprüche 1 bis 21 zur Auswahl oder/und Optimierung von Hybridisierungssonden.

26. Verwendung des Verfahren nach einem der Ansprüche 1 bis 21 zur Diagnostik.

27. Verwendung des Verfahrens nach einem der Ansprüche 1 bis 21 in der Expressionsanalyse für die Auswahl einer Subpopulation an Genen.

28. Verwendung des Verfahrens nach einem der Ansprüche 1 bis 21 zur Auswahl oder/und Optimierung von Fängersonden.

29. Verwendung des Verfahrens nach einem der Ansprüche 1 bis 21 zur Auswahl oder/und Optimierung von Antisense-Oligonukleotiden.

30. Verwendung des Verfahrens nach einem der Ansprüche 1 bis 21 zur Auswahl oder/und Optimierung von funktionellen Nukleinsäuren wie Ribozymen.

31. Verwendung des Verfahrens nach einem der Ansprüche 1 bis 21 zur Unterstützung oder/und Beschleunigung von Auswahlverfahren bei Selektionsprozessen wie Phage Display.

## Claims

1. Method for determining analytes in a sample comprising the steps:
(a) carrying out a first determination cycle comprising:
(i) providing a closed support with a surface on an interior structure which comprises immobilized receptors on a plurality of predetermined zones, where the receptors in individual zones on the reaction support each have a different analyte specificity,
(ii) contacting the sample containing the analytes to be determined with the support under conditions with which binding is possible between the analytes to be determined and receptors specific therefor on the support, and
(iii) identifying those predetermined zones on the support onto which binding has taken place in step (ii),
(b) carrying out a subsequent determination cycle comprising:
(i) providing another closed support with a surface on an interior structure which comprises immobilized receptors on a plurality of predetermined zones, where the receptors in individual zones on the reaction support each have a different analyte specificity, where the receptors selected for the other support have been observed in a preceding cycle to be associated with a predetermined characteristic signal, which is characteristic for the binding of the analyte to a specific receptor, and where the selected receptors or/and the conditions of the receptor-analyte binding are changed by comparison with a preceding determination cycle,
(ii) repeating step (a) (ii) with the other support and
(iii) repeating step (a) (iii) with the other support and
(c) where appropriate carrying out one or more further subsequent determination cycles in each case selecting and changing the receptors as in step (b) (i) until sufficient information is available about the analytes to be determined or/and until the signal meets a predetermined criterion after the binding event has occurred, the carrying out of determination cycles including the integrated production and evaluation of a support in one instrument.

2. Method according to Claim 1,
**characterized in that**
nucleic acid analytes are determined.

3. Method according to Claim 2,
**characterized in that**
the nucleic acid analytes are selected from double-stranded DNA, single-stranded DNA and RNA.

4. Method according to Claim 2 or 3,
**characterized in that**
the nucleic acid analytes are fragmented sequence-specifically or/and non-sequence-specifically before the contacting with the support.

5. Method according to Claim 4,
**characterized in that**
nucleic acid fragments with a predetermined distribution of lengths are generated by the fragmentation and, where appropriate, a subsequent fractionation by length.

6. Method according to Claim 4 or 5,
**characterized in that**
nucleic acid fragments with an essentially homogeneous distribution of lengths are generated.

7. Method according to any of the preceding claims,
**characterized in that**
the analytes carry labelling groups.

8. Method according to Claim 7,
**characterized in that**
the labelling groups can be detected optically.

9. Method according to Claim 7 or 8,
**characterized in that**
fluorescent labels or/and metal particle labels are used.

10. Method according to any of the preceding claims,
**characterized in that**
the receptors are selected from polymeric probes.

11. Method according to Claim 10,
**characterized in that**
the change in the receptors comprises a change in the probe sequence.

12. Method according to Claim 10 or 11,
**characterized in that**
the change comprises an extension of the probe sequence.

13. Method according to Claim 10 or 11,
**characterized in that**
the change comprises a variation in the probe sequence.

14. Method according to any of the preceding claims,
**characterized in that**
the change comprises a variation of the position or/and density of receptors on the support surface.

15. Method according to any of the preceding claims,
**characterized in that**
the change comprises a variation in the nature of the coupling of receptors on the support surface.

16. Method according to Claim 15,
**characterized in that**
linker molecules used to couple the receptors are varied.

17. Method according to any of the preceding claims,
**characterized in that**
the change comprises a variation of the conditions for binding between analyte and receptor.

18. Method according to Claim 17,
**characterized in that**
the hybridization conditions are varied in the case of nucleic acid analytes.

19. Method according to any of the preceding claims,
**characterized in that**
the change comprises a variation of the synthesis conditions for constructing the receptor on the support surface.

20. Method according to any of Claims 1 to 14,
**characterized by**
the change comprising a variation of the site geometry, in particular the size of the sites.

21. Method according to any of Claims 1 to 19,
**characterized in that**
the change comprises an empirical selection or specific selection of a receptor library.

22. The use of the method according to any of Claims 1 to 21 for differential expression analysis.

23. The use of the method according to any of Claims 1 to 21 for differential genome analysis.

24. The use according to Claim 23 for identifying chromosomal polymorphisms or aberrations.

25. The use of the method according to any of Claims 1 to 21 for the selection or/and optimization of hybridization probes.

26. The use of the method according to any of Claims 1 to 21 for diagnosis.

27. The use of the method according to any of Claims 1 to 21 in expression analysis for the selection of a subpopulation of genes.

28. The use of the method according to any of Claims 1 to 21 for the selection or/and optimization of capture probes.

29. The use of the method according to any of Claims 1 to 21 for the selection or/and optimization of antisense oligonucleotides.

30. The use of the method according to any of Claims 1 to 21 for the selection or/and optimization of functional nucleic acids such as ribozymes.

31. The use of the method according to any of Claims 1 to 21 for assisting or/and speeding up the selection methods in selection processes such as phage display.

## Revendications

1. Procédé pour déterminer des analytes dans une sonde comprenant les étapes consistant à :
(a) exécuter un premier cycle de détermination comprenant :
(i) la préparation d'un support fermé avec une surface située sur une structure interne qui contient des récepteurs immobilisés en une pluralité de zones prédéterminées, les récepteurs dans les différentes zones sur le support de réaction présentant à chaque fois une spécificité d'analyte différente,
(ii) la mise en contact de la sonde, qui contient des analytes à déterminer, avec le support dans des conditions sous lesquelles peut avoir lieu sur le support une liaison entre les analytes à déterminer et les récepteurs spécifiques pour cela, et
(iii) l'identification des zones prédéterminées sur le support, sur lesquelles a lieu une liaison à l'étape (ii),
(b) exécuter un cycle de détermination suivant comprenant :
(i) la préparation d'un autre support fermé avec une surface située sur une structure interne qui contient des récepteurs immobilisés en une pluralité de zones prédéterminées, les récepteurs dans les différentes zones sur le support de réaction présentant à chaque fois une spécificité d'analyte différente, des récepteurs étant sélectionnés pour l'autre support, pour lesquels il a été observé, dans un cycle précédent, un signal caractéristique prédéterminé qui est caractéristique de la liaison de l'analyte à un récepteur spécifique, et les récepteurs sélectionnés ou/et les conditions de la liaison récepteur-analyte étant modifiés par rapport à un cycle de détermination précédent,
(ii) la répétition de l'étape (a) (ii) avec l'autre support et
(iii) la répétition de l'étape (a) (iii) avec l'autre support
et
(c) exécuter, le cas échéant, un ou plusieurs autres cycles de détermination suivants à chaque fois avec sélection et modification des récepteurs selon l'étape (b) (i) jusqu'à ce que des informations suffisantes sur les analytes à déterminer soient présentes ou/et jusqu'à ce que le signal après l'événement de liaison réussi corresponde à un critère prédéterminé, moyennant quoi, l'exécution de cycles de détermination comprend la fabrication et l'analyse intégrées d'un support dans un appareil.

2. Procédé selon la revendication 1, **caractérisé en ce que** l'on détermine des analytes d'acide nucléique.

3. Procédé selon la revendication 2, **caractérisé en ce que** les analytes d'acide nucléique sont sélectionnés à partir d'ADN bicaténaires, d'ADN et d'ARN unicaténaires.

4. Procédé selon la revendication 2 ou 3, **caractérisé en ce que** les analytes d'acide nucléique avant la mise en contact avec le support sont spécifiques à une séquence ou/et sont fragmentés spécifiquement à une séquence.

5. Procédé selon la revendication 4, **caractérisé en ce que** des fragments d'acide nucléique ayant une répartition en longueur prédéterminée sont générés par la fragmentation et, le cas échéant, par un fractionnement ultérieur de la longueur.

6. Procédé selon la revendication 4 ou 5, **caractérisé en ce que** les fragments d'acide nucléique sont générés avec une répartition en longueur pour l'essentiel homogène.

7. Procédé selon l'une quelconque des revendications précédentes, **caractérisé en ce que** les analytes portent des groupes de marquages.

8. Procédé selon la revendication 7, **caractérisé en ce que** les groupes de marquages peuvent être détectés optiquement.

9. Procédé selon la revendication 7 ou 8, **caractérisé en ce que** des marquages fluorescents ou/et des marquages métalliques sont employés.

10. Procédé selon l'une quelconque des revendications précédentes, **caractérisé en ce que** les récepteurs sont sélectionnés à partir de sondes polymères.

11. Procédé selon la revendication 10, **caractérisé en ce que** la modification des récepteurs comprend une modification de la séquence de la sonde.

12. Procédé selon la revendication 10 ou 11, **caractérisé en ce que** la modification comprend une prolongation de la séquence de la sonde.

13. Procédé selon la revendication 10 ou 11, **caractérisé en ce que** la modification comprend une variation dans la séquence de la sonde.

14. Procédé selon l'une quelconque des revendications précédentes, **caractérisé en ce que** la modification comprend une variation de la position ou/et de la densité des récepteurs sur la surface du support.

15. Procédé selon l'une quelconque des revendications précédentes, **caractérisé en ce que** la modification comprend une variation dans le type de couplage des récepteurs sur la surface du support.

16. Procédé selon la revendication 15, **caractérisé en ce que** des molécules liantes employées pour le couplage des récepteurs sont soumises à une variation.

17. Procédé selon l'une quelconque des revendications précédentes, **caractérisé en ce que** la modification comprend une variation des conditions de liaison entre l'analyte et le récepteur.

18. Procédé selon la revendication 17, **caractérisé en ce qu'**a lieu une variation des conditions d'hybridation au niveau des analytes d'acide nucléique.

19. Procédé selon l'une quelconque des revendications précédentes, **caractérisé en ce que** la modification comprend une variation des conditions de synthèse lors d'une mise en place du récepteur sur la surface du support.

20. Procédé selon l'une des revendications 1 à 14, **caractérisé en ce que** la modification comprend une variation de la géométrie de l'emplacement, en particulier de la taille de l'emplacement.

21. Procédé selon l'une des revendications 1 bis 19, **caractérisé en ce que** la modification comprend un choix empirique respectivement une sélection précise d'une bibliothèque de récepteurs.

22. Emploi du procédé selon l'une des revendications 1 à 21 pour une analyse d'expression différentielle.

23. Emploi du procédé selon l'une des revendications 1 à 21 pour une analyse de génome différentielle.

24. Emploi selon la revendication 23 pour identifier des polymorphismes ou des aberrations chromosomiques.

25. Emploi du procédé selon l'une des revendications 1 à 21 à des fins de choix ou/et d'optimisation de sondes d'hybridation.

26. Emploi du procédé selon l'une des revendications 1 à 21 à des fins de diagnostic.

27. Emploi du procédé selon l'une des revendications 1 à 21 dans l'analyse d'expression pour le choix d'une sous-population de gènes.

28. Emploi du procédé selon l'une des revendications 1 à 21 à des fins de choix ou/et d'optimisation de sondes de capture.

29. Emploi du procédé selon l'une des revendications 1 à 21 à des fins de choix ou/et d'optimisation d'oligonucléotides antisens.

30. Emploi du procédé selon l'une des revendications 1 à 21 à des fins de choix ou/et d'optimisation d'acides nucléiques fonctionnels tels que des ribozymes.

31. Emploi du procédé selon l'une des revendications 1 à 21 à des fins d'assistance ou/et d'accélération du procédé de sélection lors de processus de sélection tels que Phage Display.
